# EUROPÄISCHE PATENTSCHRIFT

(11) **EP 0 836 853 B1**
(45) Veröffentlichungstag und Bekanntmachung des Hinweises auf die Patenterteilung: **08.01.2003**
(21) Anmeldenummer: 97117540.1
(22) Anmeldetag: 10.10.1997
(51) Int. Cl.: A61K 38/22

(54) **Verwendung von Bradykinin-Antagonisten zur Herstellung von Arzneimitteln zur Behandlung und Prävention der Alzheimer'schen Krankheit**
Use of bradykinin antagonists for the manufacture of a medicament for treating Alzheimer's disease
Utilisation des antagonistes de bradykinin pour la fabrication d'un médicament pour le traitement de la maladie d'Alzheimer

(30) Priorität: 14.10.1996 DE 19642289
(43) Veröffentlichungstag der Anmeldung: 22.04.1998
(73) Patentinhaber: HOECHST AKTIENGESELLSCHAFT, 65929 Frankfurt am Main (DE)
(72) Erfinder: Heitsch, Holger, Dr., 55252 Mainz-Kastel (DE); Henke, Stephan, Dr., 65719 Hofheim (DE); Breipohl, Gerhard, Dr., 60529 Frankfurt (DE); Knolle, Jochen, Dr., 65830 Kriftel (DE); Wirth, Klaus, Dr., 65830 Kriftel (DE); Wiemer, Gabriele, Prof. Dr., 61476 Kronberg (DE)

(56) Entgegenhaltungen:
- EP-A- 0 370 453
- EP-A- 0 472 220
- WO-A-94/06453
- WO-A-95/07294
- BARGER ET AL.: "Role of Cyclic GMP in the Regulation of Neuronal Calcium and Survival by Secreted Forms of beta- Amyloid Precursor" J.NEUROCHEM., Bd. 64, Nr. 5, 1995, Seiten 2087-96, XP002054763
- WIRTH ET AL.: "The bradykinin B2 receptor antagonist WIN 64338 ibhibits the effect of des-Arg9-bradykinin in endothelial cells" EUR.J.PHARMACOL.- MOLECULAR PHARMACOLOGY SECTION, Bd. 288, 1994, Seiten r1-r2, XP002054764
- NITSCH ET AL.: "Regulation of Proteolytic Processing of the Amyloid beta- Protein Precursor by First Messengers" ARZNEIM.-FORSCH./DRUD DES., Bd. 45, Nr. 3a, 1995, Seiten 435-38, XP002054765

## Beschreibung

Bradykinin und verwandte Peptide sind potente Entzündung und Schmerzen erzeugende und vasoaktive, körpereigene Substanzen. Die Verwendung von Bradykinin-Antagonisten als Mittel zur Bekämpfung von Zuständen, die durch Bradykinin vermittelt, ausgelöst und unterstützt werden, ist bekannt (EP-A-0 370 453).

Nitsch et al. (Arzneim.-Forsch. (1995), Bd. 45, Seiten 435-438; Ann. N. Y. Acad. Sci, Neurobiology of Alzheimer's disease (1996), 777, 175-182) beschreiben, daß der Bradykinin-Rezeptor die Umsetzung von APP stimuliert. Ein Bradykinin-Antagonist inhibiert laut Nitsch et al. die APP-Sekretion, was positiv bei der Bekämpfung von Alzheimer angesehen wird.

Barger et al. beschreiben in J. Neurochem. (1995), Bd. 64(5), Seite 2087-2096, Fig. 2, daß APPs die Bildung von cGMP stimuliert, und daß die Bildung von APPs eine neuroprotektive Wirkung und damit einen therapeutischen Effekt in der Behandlung der Alzheimer'schen Krankheit hat.

Wirth et al. (Eur. J. Pharmacol. (1994), Molecular Pharma Sect., Bd. 288, Seiten r1-r2) beschreiben einen nicht-peptidischen Bradykinin-B2-Antagonisten, der die Produktion von cGMP stimuliert.

Überraschenderweise wurde nun gefunden, daß die nachstehend beschriebenen Bradykinin-Antagonisten geeignete Mittel zur Behandlung der Alzheimer'schen Krankheit sind. Dies betrifft sowohl die Intention, ein Fortschreiten der Krankheit zu verhindern als auch bereits aufgetretene Symptome zu behandeln. Darüberhinaus können die Bradykinin-Antagonisten auch präventiv verwendet werden, um das Entstehen der Alzheimer'schen Krankheit zu verhindern, wenn es durch geeignete diagnostische Maßnahmen in Zukunft gelingen sollte, einen späteren Ausbruch der Krankheit vorherzusagen.

Im Gegensatz zum Stand der Technik wird ein therapeutischer Effekt erzielt, indem die Freisetzung von Alzheimerpeptiden durch Inhibierung der Bradykinin-Wirkung gehemmt wird. Die für diesen Zweck verwendeten Bradykinin-Antagonisten hemmen die Produktion von cGMP, das als indirekter Indikator für eine Freisetzung von Bradykinin durch Alzheimerpeptiden (βA) dient.

Als Verbindungen eignen sich Bradykinin-Antagonisten, die die Wirkungen des Alzheimer-Proteins Amyloid (β/A4) an isolierten Endothelzellen hemmen.

Besonders geeignet sind peptidische Bradykinin-Antagonisten, unter anderem die Peptide der Formel I

Z - P - A - B - C - E - F - K - (D)Q - G - M - F' - I (I),

in welcher bedeuten:
Z
   a₁) Wasserstoff, (C₁-C₈)-Alkyl, (C₁-C₈)-Alkanoyl, (C₁-C₈)-Alkoxycarbonyl, (C₃-C₈)-Cycloalkyl, (C₄-C₉)-Cycloalkanoyl, oder (C₁-C₈)-Alkylsulfonyl,
      in welchen jeweils 1, 2 oder 3 Wasserstoffatome jedes vorgenannten Alkyl- oder Cycloalkylrestes gegebenenfalls durch 1, 2 oder 3 gleiche oder verschiedene Reste aus der Reihe
      Carboxy, NHR(1), [(C₁-C₄)-Alkyl]NR(1) oder [(C₆-C₁₀)-Aryl-(C₁-C₄)-alkyl]NR(1), wobei R(1) für Wasserstoff oder eine Urethan-Schutzgruppe steht, (C₁-C₄)-Alkyl, (C₁-C₈)-Alkylamino, (C₆-C₁₀)-Aryl-(C₁-C₄)-alkylamino, Hydroxy, (C₁-C₄)-Alkoxy, Halogen,
      Di-(C₁-C₈)-alkylamino, Di-[(C₆-C₁₀)-aryl-(C₁-C₄)]-alkylamino, Carbamoyl, Phthalimido, 1,8-Naphthalimido, Sulfamoyl, (C₁-C₄)-Alkoxycarbonyl, (C₆-C₁₄)-Aryl und (C₆-C₁₄)-Aryl-(C₁-C₅)-alkyl ersetzt sind,
      oder in welchen jeweils 1 Wasserstoffatom gegebenenfalls durch einen Rest aus der Reihe
      (C₃-C₈)-Cycloalkyl, (C₁-C₆)-Alkylsulfonyl, (C₁-C₆)-Alkylsulfinyl, (C₆-C₁₄)-Aryl-(C₁-C₄)-alkylsulfonyl, (C₆-C₁₄)-Aryl-(C₁-C₄)-alkylsulfinyl, (C₆-C₁₄)-Aryl, (C₆-C₁₄)-Aryloxy, (C₃-C₁₃)-Heteroaryl und (C₃-C₁₃)-Heteroaryloxy
      und 1 oder 2 Wasserstoffatome durch 1 oder 2 gleiche oder verschiedene Reste aus der Reihe
      Carboxy, Amino, (C₁-C₈)-Alkylamino, Hydroxy, (C₁-C₄)-Alkoxy, Halogen, Di-(C₁-C₈)-alkylamino, Carbamoyl, Sulfamoyl, (C₁-C₄)-Alkoxycarbonyl, (C₆-C₁₄)-Aryl und (C₆-C₁₄)-Aryl-(C₁-C₅)-alkyl ersetzt sind;
   a₂) (C₆-C₁₄)-Aryl, (C₇-C₁₅)-Aroyl, (C₆-C₁₄)-Arylsulfonyl, (C₃-C₁₃)-Heteroaryl, oder (C₃-C₁₃)-Heteroaroyl;
   a₃) Carbamoyl, das gegebenenfalls am Stickstoff durch (C₁-C₈)-Alkyl, (C₆-C₁₄)-Aryl oder (C₆-C₁₄)-Aryl-(C₁-C₆)-alkyl substituiert sein kann;
      wobei in den unter a₁), a₂) und a₃) definierten Resten die Aryl-, Heteroaryl-, Aroyl-, Arylsulfonyl- und Heteroaroyl-Gruppen gegebenenfalls durch 1, 2, 3 oder 4 Reste aus der Reihe
      Carboxy, Amino, Nitro, (C₁-C₈)-Alkylamino, Hydroxy, (C₁-C₆)-Alkyl, (C₁-C₆)-Alkoxy, (C₆-C₁₄)-Aryl, (C₇-C₁₅)-Aroyl, Halogen, Cyano, Di-(C₁-C₈)-alkylamino, Carbamoyl, Sulfamoyl und (C₁-C₆)-Alkoxycarbonyl substituiert sind;
P eine direkte Bindung oder einen Rest der Formel II,

   - NR(2) -(U)- CO - (II)

   worin
   R(2) Wasserstoff, Methyl oder eine Urethan-Schutzgruppe bedeutet,
   U (C₃-C₈)-Cycloalkyliden, (C₆-C₁₄)-Aryliden, (C₃-C₁₃)-Heteroaryliden, (C₆-C₁₄)-Aryl-(C₁-C₆)-alkyliden, welche gegebenenfalls substituiert sein können, oder [CHR(3)]ₙ bedeutet, wobei n 1 - 8, vorzugsweise 1 - 6, und R(3) unabhängig voneinander Wasserstoff, (C₁-C₆)-Alkyl,
   (C₃-C₈)-Cycloalkyl, (C₆-C₁₄)-Aryl oder (C₃-C₁₃)-Heteroaryl ist,
   die mit Ausnahme des Wasserstoffs jeweils gegebenenfalls durch Amino, substituiertes Amino, Amidino, substituiertes Amidino, Hydroxy, Carboxy, Carbamoyl, Guanidino, substituiertes Guanidino, Ureido, substituiertes Ureido, Mercapto, Methylmercapto, Phenyl, 4-Chlorphenyl, 4-Fluorphenyl, 4-Nitrophenyl, 4-Methoxyphenyl, 4-Hydroxyphenyl, Phthalimido, 1,8-Naphthalimido, 4-Imidazolyl, 3-Indolyl, 2-Thienyl, 3-Thienyl, 2-Pyridyl, 3-Pyridyl oder Cyclohexyl monosubstituiert sind,
   wobei substituiertes Amino bevorzugt für -N(A')-Z, substituiertes Amidino bevorzugt für -(NH=)C-NH-Z, substituiertes Guanidino bevorzugt für -N(A')-C[=N(A')]-NH-Z und substituiertes Ureido bevorzugt für -CO-N(A')-Z stehen, in denen A' unabhängig voneinander Wasserstoff oder Z bedeutet, wobei Z wie unter a₁) oder a₂) definiert ist;
   oder worin
   R(2) und R(3) zusammen mit den diese tragenden Atomen ein mono-, bi- oder tricyclisches Ringsystem mit 2 bis 15 C-Atomen bilden;
A wie P definiert ist;
B eine basische Aminosäure in der L- oder D-Konfiguration, die in der Seitenkette substituiert sein kann;
C eine Verbindung der Formel III a oder III b 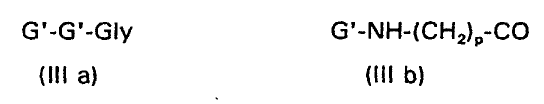 worin
   p 2 bis 8 ist, und
   G' unabhängig voneinander einen Rest der Formel IV

   -NR(4)-CHR(5)-CO- (IV)

   worin
   R(4) und R(5) zusammen mit den diese tragenden Atomen ein heterocyclisches mono-, bi- oder tricyclisches Ringsystem mit 2 bis 15 C-Atomen bilden;
E den Rest einer neutralen, sauren oder basischen, aliphatischen oder alicyclisch-aliphatischen Aminosäure;
F den Rest einer neutralen, sauren oder basischen, aliphatischen oder aromatischen Aminosäure, die in der Seitenkette substituiert sein kann, oder eine direkte Bindung;
(D)Q D-Tic, D-Phe, D-Oic, D-Thi oder D-Nal, die gegebenenfalls durch Halogen, Methyl oder Methoxy substituiert sein können oder einen Rest der nachstehenden Formel (V) 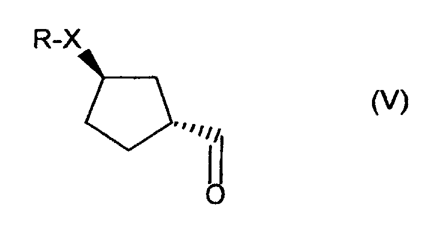 worin
   X für Sauerstoff, Schwefel oder eine direkte Bindung steht;
   R Wasserstoff, (C₁-C₈)-Alkyl, (C₃-C₈)-Cycloalkyl, (C₆-C₁₄)-Aryl, (C₆-C₁₄)-Aryl-(C₁-C₄)-alkyl, wobei der Alicyclus gegebenenfalls durch Halogen, Methyl oder Methoxy substituiert sein kann;
G wie G' oben definiert ist oder eine direkte Bindung;
F' wie F definiert ist, einen Rest -NH-(CH₂)_{q}-, mit q = 2 bis 8, oder, falls G keine direkte Bindung bedeutet, eine direkte Bindung;
I -OH, -NH₂ oder NHC₂H₅, mit der Maßgabe, daß I nicht direkt an (D)Q gebunden ist;
K den Rest -NH-(CH₂)ₓ-CO- mit x = 1 - 4 oder eine direkte Bindung, und
M wie F definiert ist,
sowie deren physiologisch verträgliche Salze.

Geeignete Bradykinin-Antagonisten und deren Herstellung sind zum Beispiel beschrieben in den Patent-Anmeldungen WO 95/07294 [Scios Nova, Pseudopeptide], WO 94/08607 [Scios Nova, Pseudopeptide], WO 94/06453 [Stewart, aliphatic amino acid in 5-position], WO 93/11789 [Nova], EP-A 552 106 [Adir], EP-A 578 521 [Adir], WO 94/19372 [Scios Nova, Cyclopeptide], EP-A 370 453 [Hoechst], EP-A 472 220 [Syntex], WO 92/18155 [Nova], WO 92/18156 [Nova], WO 92/17201 [Cortech] und WO 94/11021 [Cortech; Bradykinin-Antagonisten der Formel X(BKA)ₙ, worin X ein Bindeglied, BKA die Peptidkette eines Bradykinin-Antagonisten und n eine ganze Zahl größer als 1 ist; Bradykinin-Antagonisten der Formel X(BKA); und Bradykinin-Antagonisten der Formel (Y)(X)(BKA) mit Y gleich einem Liganden, welcher ein Antagonist oder ein Agonist für einen Nicht-Bradykinin-Rezeptor ist].

Besonders geeignet sind Peptide der Formel I, in welcher bedeuten:
Z Wasserstoff oder wie oben unter a₁), a₂) oder a₃) definiert,
P eine Bindung oder ein Rest der Formel II

   - NR(2) -(U)- CO - (II)

   mit U gleich CHR(3) und
   R(3) wie oben definiert; oder
   worin R(2) und R(3) zusammen mit den diese tragenden Atomen ein mono-, bi- oder tricyclisches Ringsystem mit 2 bis 15 C-Atomen bilden,
   R(2) gleich H oder CH₃,
A eine Bindung.

Insbesondere sind Verbindungen der Formel I bevorzugt, in der bedeuten:
Z Wasserstoff oder wie oben unter a₁), a₂) oder a₃) definiert,
P eine Bindung oder ein Rest der Formel II

   - NR(2) -(U)- CO - (II)

   mit U gleich CHR(3) und
   R(3) unabhängig voneinander Wasserstoff, (C₁-C₆)-Alkyl, (C₃-C₈)-Cycloalkyl, (C₆-C₁₄)-Aryl, (C₃-C₁₃)-Heteroaryl, die mit Ausnahme des Wasserstoffs jeweils gegebenenfalls durch
   Amino, substituiertes Amino, Hydroxy, Carboxy, Carbamoyl, Guanidino, substituiertes Guanidino, Ureido, Mercapto, Methylmercapto, Phenyl, 4-Chlorphenyl, 4-Fluorphenyl, 4-Nitrophenyl, 4-Methoxyphenyl, 4-Hydroxyphenyl, Phthalimido, 4-Imidazolyl, 3-Indolyl, 2-Thienyl, 3-Thienyl, 2-Pyridyl, 3-Pyridyl oder Cyclohexyl monosubstituiert sind,
   wobei substituiertes Amino bevorzugt für -N(A')-Z und substituiertes Guanidino bevorzugt für -N(A')-C[=N(A')]-NH-Z stehen, in denen A' unabhängig voneinander Wasserstoff oder Z bedeutet, wobei Z wie unter a₁) oder a₂) definiert ist; oder
   worin R(2) und R(3) zusammen mit den diese tragenden Atomen ein mono-, bi- oder tricyclisches Ringsystem mit 2 bis 15 C-Atomen bilden,
   R(2) gleich H oder CH₃;
A eine Bindung;
(D)Q D-Tic.

Bevorzugt geeignet sind:
H-D-Arg-Arg-Pro-Hyp-Gly-Thi-Ser-D-Tic-Oic-Arg-OH (HOE 140) para-Guanidobenzoyl-Arg-Pro-Hyp-Gly-Thi-Ser-D-Tic-Oic-Arg-OH H-D-Arg-Arg-Pro-Hyp-Gly-Phe-Ser-D-HypE(transpropyl)-Oic-Arg-OH H-D-Arg-Arg-Pro-Hyp-Gly-Cpg-Ser-D-Cpg-Cpg-Arg-OH H-D-Arg-Arg-Pro-Pro-Gly-Thi-Ser-D-Tic-Oic-Arg-OH H-Arg(Tos)-Pro-Hyp-Gly-Thi-Ser-D-Tic-Oic-Arg-OH H-Arg(Tos)-Pro-Hyp-Gly-Phe-Ser-D-Tic-Oic-Arg-OH H-D-Arg-Arg-Pro-Hyp-Gly-Phe-Ser-D-Tic-Oic-Arg-OH Fmoc-D-Arg-Arg-Pro-Hyp-Gly-Thi-Ser-D-Tic-Oic-Arg-OH Fmoc-Aoc-D-Arg-Arg-Pro-Hyp-Gly-Thi-Ser-D-Tic-Oic-Arg-OH Fmoc-e-Aminocaproyl-D-Arg-Arg-Pro-Hyp-Gly-Thi-Ser-D-Tic-Oic-Arg-OH Benzoyl-D-Arg-Arg-Pro-Hyp-Gly-Thi-Ser-D-Tic-Oic-Arg-OH Cyclohexylcarbonyl-D-Arg-Arg-Pro-Hyp-Gly-Thi-Ser-D-Tic-Oic-Arg-OH Fmoc-Aeg(Fmoc)-D-Arg-Arg-Pro-Hyp-Gly-Thi-Ser-D-Tic-Oic-Arg-OH Fmoc-Aeg(Fmoc)-Arg-Pro-Hyp-Gly-Thi-Ser-D-Tic-Oic-Arg-OH Indol-3-yl-acetyl-D-Arg-Arg-Pro-Hyp-Gly-Thi-Ser-D-Tic-Oic-Arg-OH Dibenzylacetyl-D-Arg-Arg-Pro-Hyp-Gly-Thi-Ser-D-Tic-Oic-Arg-OH und deren physiologisch verträglichen Salze.

Besonders geeignet sind
H-D-Arg-Arg-Pro-Hyp-Gly-Thi-Ser-D-Tic-Oic-Arg-OH (HOE 140), para-Guanidobenzoyl-Arg-Pro-Hyp-Gly-Thi-Ser-D-Tic-Oic-Arg-OH und deren physiologisch verträglichen Salze.

Ganz besonders geeignet ist H-D-Arg-Arg-Pro-Hyp-Gly-Thi-Ser-D-Tic-Oic-Arg-OH (HOE 140) und dessen physiologisch verträglichen Salze.

Die Anwendung kann enteral, parenteral - wie z. B. subkutan, i. m. oder i. v. -, nasal, rektal oder per Inhalation erfolgen. Die Dosierung des Wirkstoffs hängt vom Körpergewicht, Alter und von der Applikationsart ab.

Die pharmazeutischen Präparate der vorliegenden Erfindung werden in an sich bekannten Lösungs-, Misch-, Granulier- , Tabletten- oder Dragierverfahren hergestellt.

Zur parenteralen Applikation werden die aktiven Verbindungen oder deren physiologisch verträgliche Salze, gewünschtenfalls mit den pharmazeutisch üblichen Hilfsstoffen, beispielsweise zur Isotonisierung oder pH-Einstellung sowie Lösungsvermittler, Emulgatoren, oder anderen Hilfsstoffen, in Lösung, Suspension oder Emulsion gebracht.

Für die beschriebenen Arzneistoffe ist auch der Einsatz von injizierbaren Retardzubereitungen zur subkutanen oder intramuskulären Anwendung sinnvoll. Als Arzneiformen können z. B. ölige Kristallsuspensionen, Mikrokapseln, Mikropartikel, Nanopärtikel oder Implantate verwendet werden, wobei die letzteren aus gewebeverträglichen Polymeren, insbesondere bioabbaubaren Polymeren, wie z. B. auf der Basis von

Polymilchsäure-Polyglykolsäure-Copolymeren aufgebaut sein können. Andere denkbare Polymere sind Polyamide, Polyester, Polyacetate oder Polysaccharide.

Für die orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesia, Magnesiumcarbonat, Kaliumphosphat, Milchzucker, Glucose, Magnesiumstearylfumarat oder Stärke, insbesondere Maisstärke verwendet werden. Dabei kann die Zubereitung von festen Arzneiformen sowohl als Trocken- und Feuchtgranulat erfolgen. Als ölige Trägerstoffe oder Lösungsmittel kommen beispielsweise pflanzliche oder tierische Öle in Betracht, wie Sonnenblumenöl und Lebertran.

Auch orale Retardzubereitungen oder Zubereitungen mit magensaftresistenten Überzügen sind denkbar. Retardzubereitungen können auf der Basis von Fett-, Wachs- oder Polymereinbettungen aufgebaut sein. Möglich sind hierbei auch Mehrschicht- oder Manteltabletten oder Pellets.

Für die beschriebenen Arzneistoffe ist auch eine Applikation auf Schleimhäute zur Erzielung systemisch wirksamer Spiegel sinnvoll. Dies betrifft die Möglichkeit der intranasalen, inhalativen und rektalen Anwendung.

Für die intranasale Anwendungsform werden die Verbindungen mit den dafür üblichen Zusatzstoffen wie Stabilisatoren oder inerten Verdünnungsmitteln vermischt und durch übliche Methoden in geeignete Darreichungsformen gebracht, wie Pulver, wäßrige, alkoholische oder ölige Suspensionen oder wäßrige, alkoholische oder ölige Lösungen. Wäßrigen intranasalen Zubereitungen können Chelatbildner, wie Ethylendiamin-N,N,N',N'-tetraessigsäure und Puffer wie Essigsäure, Phosphorsäure, Citronensäure, Weinsäure und deren Salze zugefügt werden. Mehrfachdosenbehälter enthalten Konservierungsmittel wie Benzalkoniumchlorid, Chlorbutanol, Chlorhexidin, Sorbinsäure, Benzoesäure, PHB-Ester oder Organoquecksilberverbindungen.
Die Applikation der Nasallösungen kann mittels Dosierzerstäuber erfolgen oder als Nasaltropfen mit viskositätserhöhendem Anteil bzw. Nasengels oder Nasencremes.
Für die inhalative Anwendung können Vernebler oder Druckgas-Packungen unter Verwendung inerter Trägergase benutzt werden.
Zur Applikation von Pulvern zur nasalen oder pulmonalen Inhalation sind spezielle Applikatoren erforderlich.

Die wirksame Dosis beträgt mindestens 0.001 mg/kg/Tag, vorzugsweise mindestens 0.01 mg/kg/Tag, höchstens 5 mg/kg/Tag, vorzugsweise 0.03 bis 1 mg//kg/Tag Körpergewicht in Abhängigkeit vom Schweregrad der Symptomatik, bezogen auf einen Erwachsenen von 75 kg Körpergewicht.

Die für Aminosäuren verwendeten Abkürzungen entsprechen dem in der Peptidchemie üblichen drei Buchstaben-Code wie er in Europ. J. Biochem 138,9 (1984) beschrieben ist. Weitere verwendete Abkürzungen sind nachfolgend aufgelistet:
- Aeg: N-(2-Aminoethyl)glycin
- Cpg: Cyclopentylglycyl
- Fmoc: 9-Fluorenylmethyloxycarbonyl
- Nal: 2-Naphthylalanyl
- Oic: cis,endo-Octahydroindol-2-carbonyl
- Thi: 2-Thienylalanyl
- Tic: 1,2,3,4-Tetrahydroisochinolin-3-ylcarbonyl

### Beispiele:

Wirkung der Verbindungen der Formel (I) auf die durch das Alzheimer-Protein β/A4 stimulierte cGMP-Produktion in Endothelzellkulturen

### Testsysteme:

Bovine Aorten-Endothelzell-Kulturen (BAECs = bovine aortic endothelial cells), intravaskuläre koronare Ratten-Endothelzell-Kulturen (RMCECs =rat microvascular coronary endothelial cells) und humane Nabelschnur-Endothelzell-Kulturen (HUVECs = human umbilical vein endothelial cells)

### Methode:

Bestimmung des Einflusses von Bradykinin-Antagonisten der Formel (I) auf die durch Gabe von 0.1 und 1 µmol/l des Alzheimer-Proteins β/A4 stimulierte Produktion von cGMP in Endothelzell-Kulturen verschiedener Species und Organen.
cGMP: zyklisches Guanosinmonophosphat

Es ist hinreichend gezeigt worden, daß Endothelzellen ein geeignetes Testsystem zum Nachweis einer Wirkung und Freisetzung von Bradykinin darstellen (G. Wiemer et al., Hypertension 1991; 18:558-563). An Endothelzellen führt Bradykinin zur Erhöhung der Produktion von cGMP, welches mittels eines Radioimmunassays bestimmt wird. Erhöhung der Bildung von cGMP durch Bradykinin ist ein Indikator für eine Freisetzung von NO (Stickstoffmonoxid) aus Endothelzellen.

### Versuch:

Stimulation der cGMP-Produktion durch βA(1-40) und inhibitorischer Effekt des Bradykinin-Antagonisten HOE 140 (10⁻⁷ mol/l) in 3 verschiedenen Arten von Endothel-Zellen:

| | pmol/mg Protein | | |
|---|---|---|---|
| Endothel Zell-Typ | BAECs | RMCECs | HUVECs |
| Basale cGMP-Produktion | 2.2±0.35 | 0.2±0.07 | 4.75±0.4 |
| BK 10⁻⁸ mol/l | 8.8±0.34 | 1.13±0.2 | 11.46±2 |
| BK 10⁻⁸ mol/l + HOE 140 | 2.2±0.2 | 0.22±0.02 | 3.45±0.45 |
| βA (1-40) 10⁻⁷ mol/l | 5.9±0.23 | 0.59±0.07 | 14.07±1.6 |
| βA (1-40) 10⁻⁷ mol/l + HOE 140 | 2.2±0.28 | 0.34±0.02 | 4.0±0.46 |
| βA (1-40) 10⁻⁶ mol/l | 4.6±0.13 | 0.74±0.09 | 15.3±1.9 |
| βA (1-40) 10⁻⁶ mol/l + HOE 140 | 2.2±0.2 | 0.32±0.05 | 5.3±0.49 |

### Ergebnis:

Die gleichzeitige Inkubation der o.a. Zellkulturen verschiedener Species und Organe mit HOE 140 als repräsentatives Beispiel der Verbindungen der Formel (I) in einer Konzentration von 0.1 µmol/l verhindert die vom β/A4-Protein ausgelöste Stimulierung der Produktion von cGMP.

### Bewertung:

Der durchgeführte Versuch zeigt an, daß die Wirkung des Alzheimer-Proteins β/A4 auf die Produktion von cGMP über eine Bindung von Bradykinin an seine Zellrezeptoren vermittelt ist.
Endothelzellkulturen dienen hierbei als Indikator einer Wirkung von β/A4, die über Bradykinin vermittelt ist. Die Endothelzellen sind dabei jedoch nicht nur das Indikatorsystem für eine Wirkung über Bradykinin-Rezeptoren, sondern auch das Effektororgan bei der Alzheimer'schen Krankheit. Endothelzellen sind Bestandteile der Blutgefäße und kleiden diese aus. Die Blutgefäße selbst sind von Ablagerungen des Alzheimer-Proteins Amyloid (β/A4) bei der Alzheimer'schen Krankheit neben neuronalem Gewebe stark betroffen. Endothelzellen sind verantwortlich für eine durch Bradykinin ausgelöste Steigerung der Durchlässigkeit der Bluthirnschranke.

Zunehmend wird die Bedeutung einer lokalisierten Entzündung für die destruktiven Veränderungen im Gehirn von Patienten mit Alzheimer'scher Krankheit erkannt. Entzündliche Veränderungen führen zur Chronifizierung und zur fortschreitenden Destruktion des Gehirns und damit schweren Demenz (J. Rogers, Inflammation as a pathogenic mechanism in Alzheimer's disease, Arzneimittelforschung 1995; 45 (3A),439-442). Es war bisher nicht bekannt, daß Bradykinin, ein stark entzündlicher Mediator in der Peripherie, eine Rolle bei Alzheimer'scher Krankheit spielen könnte. Dies ist darauf zurückzuführen, daß es keine Vorstellungen zur Freisetzung von Bradykinin im Gehirn von Patienten mit Alzheimer'scher Krankheit gab. Die inaktiven hochmolekularen Vorstufen, aus denen Bradykinin freigesetzt wird, können nämlich wegen der geringen Durchlässigkeit der Bluthirnschranke nicht ohne weiteres ins Gehirn (neuronale Gewebe) gelangen.

Unsere Untersuchungen zeigen, daß das Alzheimer Protein β/A4 aus dem Endothel von Gefäßwänden Bradykinin freisetzen kann. Dem Alzheimer Protein β/A4 werden die wesentlichen pathologischen Veränderungen der Alzheimer Krankheit zugeschrieben (Joachim, C.L.; Selkoe, D.J., The seminal role of betaamyloid in the pathogenesis of Alzheimer disease, Alzheimer-DIS-ASSOC-Disord. 1992, Spring; 6(1): 7-34). Ist einmal die Freisetzung des entzündlich wirksamen Bradykinins durch einen Mechanismus gezeigt, der spezifisch für die Alzheimer'sche Krankheit ist, wird Bradykinin zum pathophysiologischen Faktor ersten Ranges, über welchen das Alzheimer Protein seine destruktive Wirkung vermitteln kann. Dies gilt vor allem in Hinblick auf die Entzündung, deren Bedeutung für die destruktiven Veränderungen zunehmend erkannt wird, denn Bradykinin ist eine der stärksten entzündlich wirksamen körpereigenen Substanzen. Neben der entzündlichen Wirkung besitzt Bradykinin noch zwei weitere Eigenschaften, durch welche es zu den destruktiven Veränderungen bei Alzheimer'scher Krankheit beitragen kann. Bradykinin stimuliert ZNS-Neurone. Bei starker Stimulation führt dies zur Kalziumüberladung der betroffenen Zellen mit nachfolgendem Zelltod. Bei mäßiger Stimulierung wird Bradykinin nur zum falschen Transmitter, welcher Neurone inadäquat stimuliert. Eine solche inadäquate Stimulierung von Neuronen, die eigentliche gar nicht erregt werden sollten, kann den Prozeß der Informationsverarbeitung im Gehirn empfindlich stören und zu den typischen Hirnleistungsstörungen beitragen, wobei der letztere Mechanismus, ausgelöst durch mäßige Stimulierung, reversibel erscheint. Als vasoaktiver Mediator erhöht Bradykinin bekanntermaßen die Durchlässigkeit der Bluthirnschranke. Dies führt dazu, daß die Vorstufen von Bradykinin erst von den Blutgefäßen ins Gehirn gelangen können, um dort ihre zerstörerische Wirkung zu entfalten.

Somit sind Bradykininantagonisten der Formel (I) geeignet für die therapeutische und präventive Behandlung der Alzheimer'schen Krankheit.

## Patentansprüche

1. Verwendung eines Bradykinin-Antagonisten der Formel I
Z - P - A - B - C - E - F - K - (D)Q - G - M - F' - I (I),
in welcher bedeuten:
Z
a₁) Wasserstoff, (C₁-C₈)-Alkyl, (C₁-C₈)-Alkanoyl, (C₁-C₈)-Alkoxycarbonyl, (C₃-C₈)-Cycloalkyl, (C₄-C₉)-Cycloalkanoyl, oder (C₁-C₈)-Alkylsulfonyl,
in welchen jeweils 1, 2 oder 3 Wasserstoffatome jedes vorgenannten Alkyl- oder Cycloalkylrestes gegebenenfalls durch 1, 2 oder 3 gleiche oder verschiedene Reste aus der Reihe
Carboxy, NHR(1), [(C₁-C₄)-Alkyl]NR(1) oder [(C₆-C₁₀)-Aryl-(C₁-C₄)-alkyl]NR(1), wobei R(1) für Wasserstoff oder eine Urethan-Schutzgruppe steht, (C₁-C₄)-Alkyl, (C₁-C₈)-Alkylamino, (C₆-C₁₀)-Aryl-(C₁-C₄)-alkylamino, Hydroxy, (C₁-C₄)-Alkoxy, Halogen, Di-(C₁-C₈)-alkylamino, Di-[(C₆-C₁₀)-aryl-(C₁-C₄)]-alkylamino, Carbamoyl, Phthalimido, 1,8-Naphthalimido, Sulfamoyl, (C₁-C₄)-Alkoxycarbonyl, (C₆-C₁₄)-Aryl und (C₆-C₁₄)-Aryl-(C₁-C₅)-alkyl ersetzt sind,
oder in welchen jeweils 1 Wasserstoffatom gegebenenfalls durch einen Rest aus der Reihe
(C₃-C₈)-Cycloalkyl, (C₁-C₆)-Alkylsulfonyl, (C₁-C₆)-Alkylsulfinyl, (C₆-C₁₄)-Aryl-(C₁-C₄)-alkylsulfonyl, (C₆-C₁₄)-Aryl-(C₁-C₄)-alkylsulfinyl, (C₆-C₁₄)-Aryl, (C₆-C₁₄)-Aryloxy, (C₃-C₁₃)-Heteroaryl und (C₃-C₁₃)-Heteroaryloxy
und 1 oder 2 Wasserstoffatome durch 1 oder 2 gleiche oder verschiedene Reste aus der Reihe
Carboxy, Amino, (C₁-C₈)-Alkylamino, Hydroxy, (C₁-C₄)-Alkoxy, Halogen, Di-(C₁-C₈)-alkylamino, Carbamoyl, Sulfamoyl, (C₁-C₄)-Alkoxycarbonyl, (C₆-C₁₄)-Aryl und (C₈-C₁₄)-Aryl-(C₁-C₅)-alkyl ersetzt sind;
a₂) (C₆-C₁₄)-Aryl, (C₇-C₁₅)-Aroyl, (C₆-C₁₄)-Arylsulfonyl, (C₃-C₁₃)-Heteroaryl, oder (C₃-C₁₃)-Heteroaroyl;
a₃) Carbamoyl, das gegebenenfalls am Stickstoff durch (C₁-C₈)-Alkyl, (C₆-C₁₄)-Aryl oder (C₆-C₁₄)-Aryl-(C₁-C₅)-alkyl substituiert sein kann;
wobei in den unter a₁), a₂) und a₃) definierten Resten die Aryl-, Heteroaryl-, Aroyl-, Arylsulfonyl- und Heteroaroyl-Gruppen gegebenenfalls durch 1, 2, 3 oder 4 Reste aus der Reihe
Carboxy, Amino, Nitro, (C₁-C₈)-Alkylamino, Hydroxy, (C₁-C₆)-Alkyl, (C₁-C₆)-Alkoxy, (C₆-C₁₄)-Aryl, (C₇-C₁₅)-Aroyl, Halogen, Cyano, Di-(C₁-C₈)-alkylamino, Carbamoyl, Sulfamoyl und (C₁-C₆)-Alkoxycarbonyl substituiert sind;
P eine direkte Bindung oder einen Rest der Formel II,
- NR(2) -(U)- CO - (II)
worin
R(2) Wasserstoff, Methyl oder eine Urethan-Schutzgruppe bedeutet,
U (C₃-C₈)-Cycloalkyliden, (C₆-C₁₄)-Aryliden, (C₃-C₁₃)-Heteroaryliden, (C₆-C₁₄)-Aryl-(C₁-C₆)-alkyliden, welche gegebenenfalls substituiert sein können, oder [CHR(3)]ₙ bedeutet, wobei n 1 - 8, vorzugsweise 1 - 6, und R(3) unabhängig voneinander Wasserstoff, (C₁-C₆)-Alkyl,
(C₃-C₈)-Cycloalkyl, (C₆-C₁₄)-Aryl oder (C₃-C₁₃)-Heteroaryl ist,
die mit Ausnahme des Wasserstoffs jeweils gegebenenfalls durch Amino, substituiertes Amino, Amidino, substituiertes Amidino, Hydroxy, Carboxy, Carbamoyl, Guanidino, substituiertes Guanidino, Ureido, substituiertes Ureido, Mercapto, Methylmercapto, Phenyl, 4-Chlorphenyl, 4-Fluorphenyl, 4-Nitrophenyl, 4-Methoxyphenyl, 4-Hydroxyphenyl, Phthalimido, 1,8-Naphthalimido, 4-Imidazolyl, 3-Indolyl, 2-Thienyl, 3-Thienyl, 2-Pyridyl, 3-Pyridyl oder Cyclohexyl monosubstituiert sind,
oder worin
R(2) und R(3) zusammen mit den diese tragenden Atomen ein mono-, bi- oder tricyclisches Ringsystem mit 2 bis 15 C-Atomen bilden;
A wie P definiert ist;
B eine basische Aminosäure in der L- oder D-Konfiguration, die in der Seitenkette substituiert sein kann;
C eine Verbindung der Formel III a oder III b 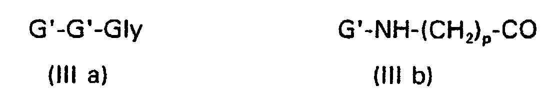 worin
p 2 bis 8 ist, und
G' unabhängig voneinander einen Rest der Formel IV
-NR(4)-CHR(5)-CO- (IV)
worin
R(4) und R(5) zusammen mit den diese tragenden Atomen ein heterocyclisches mono-, bi- oder tricyclisches Ringsystem mit 2 bis 15 C-Atomen bilden;
E den Rest einer neutralen, sauren oder basischen, aliphatischen oder alicyclisch-aliphatischen Aminosäure;
F unabhängig voneinander den Rest einer neutralen, sauren oder basischen, aliphatischen oder aromatischen Aminosäure, die in der Seitenkette substituiert sein kann, oder eine direkte Bindung;
(D)Q D-Tic, D-Phe, D-Oic, D-Thi oder D-Nal, die gegebenenfalls durch Halogen, Methyl oder Methoxy substituiert sein können oder einen Rest der nachstehenden Formel (V) 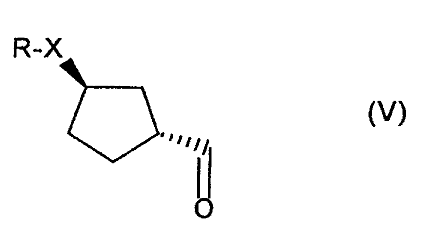 worin
X für Sauerstoff, Schwefel oder eine direkte Bindung steht;
R Wasserstoff, (C₁-C₈)-Alkyl, (C₃-C₈)-Cycloalkyl, (C₆-C₁₄)-Aryl, (C₆-C₁₄)-Aryl-(C₁-C₄)-alkyl, wobei Cycloalkyl gegebenenfalls durch Halogen, Methyl oder Methoxy substituiert sein kann;
G wie G' oben definiert ist oder eine direkte Bindung;
F' wie F definiert ist, einen Rest -NH-(CH₂)_{q}-, mit q = 2 bis 8, oder, falls G keine direkte Bindung bedeutet, eine direkte Bindung;
I -OH, -NH₂ oder NHC₂H₅, mit der Maßgabe, daß I nicht direkt an (D)Q gebunden ist;
K den Rest -NH-(CH₂)ₓ-CO- mit x = 1 - 4 oder eine direkte Bindung, und
M wie F definiert ist,
sowie deren physiologisch verträgliche Salze
zur Herstellung von Arzneimitteln zur Behandlung und zur Prävention der Alzheimer'schen Krankheit.

2. Verwendung eines Bradykinin-Antagonisten der Formel I nach Anspruch 1, worin
Z Wasserstoff oder wie unter a₁), a₂) oder a₃) definiert ist,
P eine Bindung oder ein Rest der Formel II ist,
- NR(2) -(U)- CO - (II)
mit U gleich CHR(3) und
R(3) wie oben definiert; oder
worin R(2) und R(3) zusammen mit den diese tragenden Atomen ein mono-, bi- oder tricyclisches Ringsystem mit 2 bis 15 C-Atomen bilden,
R(2) gleich H oder CH₃ und
A eine Bindung ist.

3. Verwendung eines Bradykinin-Antagonisten der Formel I nach Anspruch 1, worin
Z Wasserstoff oder wie unter a₁), a₂) oder a₃) definiert ist,
P eine Bindung oder ein Rest der Formel II ist,
- NR(2) -(U)- CO - (II)
mit U gleich CHR(3) und R(3) unabhängig voneinander Wasserstoff, (C₁-C₆)-Alkyl, (C₃-C₈)-Cycloalkyl, (C₆-C₁₄)-Aryl, (C₃-C₁₃)-Heteroaryl, die mit Ausnahme des Wasserstoffs jeweils gegebenenfalls durch Amino, substituiertes Amino, Hydroxy, Carboxy, Carbamoyl, Guanidino, substituiertes Guanidino, Ureido, Mercapto, Methylmercapto, Phenyl, 4-Chlorphenyl, 4-Fluorphenyl, 4-Nitrophenyl, 4-Methoxyphenyl, 4-Hydroxyphenyl, Phthalimido, 4-Imidazolyl, 3-Indolyl, 2-Thienyl, 3-Thienyl, 2-Pyridyl, 3-Pyridyl oder Cyclohexyl monosubstituiert sind, oder worin R(2) und R(3) zusammen mit den diese tragenden Atomen ein mono-, bi- oder tricyclisches Ringsystem mit 2 bis 15 C-Atomen bilden;
R(2) gleich H oder CH₃ ist,
A eine Bindung und
(D)Q D-Tic ist.

4. Verwendung eines Bradykinin-Antagonisten nach den Ansprüchen 1 - 3, **dadurch gekennzeichnet daß** der Bradykinin-Antagonist
H-D-Arg-Arg-Pro-Hyp-Gly-Thi-Ser-D-Tic-Oic-Arg-OH (HOE 140), para-Guanidinobenzoyl-Arg-Pro-Hyp-Gly-Thi-Ser-D-Tic-Oic-Arg-OH, H-D-Arg-Arg-Pro-Hyp-Gly-Phe-Ser-D-HypE(transpropyl)-Oic-Arg-OH, H-D-Arg-Arg-Pro-Hyp-Gly-Cpg-Ser-D-Cpg-Cpg-Arg-OH, H-D-Arg-Arg-Pro-Pro-Gly-Thi-Ser-D-Tic-Oic-Arg-OH, H-Arg(Tos)-Pro-Hyp-Gly-Thi-Ser-D-Tic-Oic-Arg-OH, H-Arg(Tos)-Pro-Hyp-Gly-Phe-Ser-D-Tic-Oic-Arg-OH, H-D-Arg-Arg-Pro-Hyp-Gly-Phe-Ser-D-Tic-Oic-Arg-OH, Fmoc-D-Arg-Arg-Pro-Hyp-Gly-Thi-Ser-D-Tic-Oic-Arg-OH, Fmoc-Aoc-D-Arg-Arg-Pro-Hyp-Gly-Thi-Ser-D-Tic-Oic-Arg-OH, Fmoc- -Aminocaproyl-D-Arg-Arg-Pro-Hyp-Gly-Thi-Ser-D-Tic-Oic-Arg-OH, Benzoyl-D-Arg-Arg-Pro-Hyp-Gly-Thi-Ser-D-Tic-Oic-Arg-OH, Cyclohexylcarbonyl-D-Arg-Arg-Pro-Hyp-Gly-Thi-Ser-D-Tic-Oic-Arg-OH, Fmoc-Aeg(Fmoc)-D-Arg-Arg-Pro-Hyp-Gly-Thi-Ser-D-Tic-Oic-Arg-OH, Fmoc-Aeg(Fmoc)-Arg-Pro-Hyp-Gly-Thi-Ser-D-Tic-Oic-Arg-OH, Indol-3-yl-acetyl-D-Arg-Arg-Pro-Hyp-Gly-Thi-Ser-D-Tic-Oic-Arg-OH oder Dibenzylacetyl-D-Arg-Arg-Pro-Hyp-Gly-Thi-Ser-D-Tic-Oic-Arg-OH ist.

5. Verwendung eines Bradykinin-Antagonisten gemäß Anspruch 4, **dadurch gekennzeichnet, daß** der Bradykinin-Antagonist,
H-D-Arg-Arg-Pro-Hyp-Gly-Thi-Ser-D-Tic-Oic-Arg-OH (HOE 140) oder para-Guanidobenzoyl-Arg-Pro-Hyp-Gly-Thi-Ser-D-Tic-Oic-Arg-OH ist.

6. Verwendung eines Bradykinin-Antagonisten gemäß Anspruch 5, **dadurch gekennzeichnet, daß** der Bradykinin-Antagonist,
H-D-Arg-Arg-Pro-Hyp-Gly-Thi-Ser-D-Tic-Oic-Arg-OH (HOE 140) ist.

## Claims

1. Use of a bradykinin antagonist of the formula I
Z - P - A - B - C - E - F - K - (D)Q - G - M - F' - I (I)
in which:
Z is
a₁) hydrogen, (C₁-C₈)-alkyl, (C₁-C₈)-alkanoyl, (C₁-C₈)-alkoxycarbonyl, (C₃-C₈)-cycloalkyl, (C₄-C₉)-cycloalkanoyl or (C₁-C₈)-alkylsulfonyl,
in each of which 1, 2 or 3 hydrogen atoms of each of the above-mentioned alkyl or cycloalkyl radical is or are optionally replaced by 1, 2 or 3 identical or different radicals from the series consisting of
carboxyl, NHR(1), [(C₁-C₄)-alkyl]NR(1) or [(C₆-C₁₀)-aryl-(C₁-C₄)-alkyl]NR(1), where R(1) is hydrogen or a urethane-protecting group, (C₁-C₄)-alkyl, (C₁-C₈)-alkylamino, (C₆-C₁₀)-aryl-(C₁-C₄)-alkylamino, hydroxyl, (C₁-C₄)-alkoxy, halogen, di(C₁-C₈)-alkylamino, di[(C₆-C₁₀)-aryl-(C₁-C₄)]-alkylamino, carbamoyl, phthalimido, 1,8-naphthalimido, sulfamoyl, (C₁-C₄)-alkoxycarbonyl, (C₆-C₁₄)-aryl and (C₆-C₁₄)-aryl-(C₁-C₅)-alkyl,
or in each of which 1 hydrogen atom is optionally replaced by a radical from the series consisting of
(C₃-C₈)-cycloalkyl, (C₁-C₆)-alkylsulfonyl, (C₁-C₆)-alkylsulfinyl, (C₆-C₁₄)-aryl-(C₁-C₄)-alkylsulfonyl, (C₆-C₁₄)-aryl-(C₁-C₄)-alkylsulfinyl, (C₆-C₁₄)-aryl, (C₆-C₁₄)-aryloxy, (C₃-C₁₃)-heteroaryl and (C₃-C₁₃)-heteroaryloxy
and 1 or 2 hydrogen atoms are replaced by 1 or 2 identical or different radicals from the series consisting of
carboxyl, amino, (C₁-C₈)-alkylamino, hydroxyl, (C₁-C₄)-alkoxy, halogen, di(C₁-C₈)-alkylamino, carbamoyl, sulfamoyl, (C₁-C₄)-alkoxycarbonyl, (C₆-C₁₄)-aryl and (C₆-C₁₄)-aryl-(C₁-C₅)-alkyl;
a₂) (C₆-C₁₄)-aryl, (C₇-C₁₅)-aroyl, (C₆-C₁₄)-arylsulfonyl, (C₃-C₁₃)-heteroaryl or (C₃-C₁₃)-heteroaroyl;
a₃) carbamoyl, which may optionally be substituted on the nitrogen by (C₁-C₈)-alkyl, (C₆-C₁₄)-aryl or (C₆-C₁₄)-aryl-(C₁-C₅)-alkyl;
where the aryl, heteroaryl, aroyl, arylsulfonyl and heteroaroyl groups in the radicals defined under a₁), a₂) and a₃) are optionally substituted by 1, 2, 3 or 4 radicals from the series consisting of
carboxyl, amino, nitro, (C₁-C₈)-alkylamino, hydroxyl, (C₁-C₆)-alkyl, (C₁-C₆)-alkoxy, (C₆-C₁₄)-aryl, (C₇-C₁₅)-aroyl, halogen, cyano, di(C₁-C₈)-alkylamino, carbamoyl, sulfamoyl and (C₁-C₆)-alkoxycarbonyl;
P is a direct bond or a radical of the formula II
-NR(2)-(U)-CO- (II)
in which
R(2) is hydrogen, methyl or a urethane-protecting group,
U is (C₃-C₈)-cycloalkylidene, (C₆-C₁₄)-arylidene, (C₃-C₁₃)-heteroarylidene, (C₆-C₁₄)-aryl-(C₁-C₆)-alkylidene, which may optionally be substituted, or [CHR(3)]ₙ, where n is 1 - 8, preferably 1 - 6, and
R(3) independently of one another is hydrogen,
(C₁-C₆)-alkyl, (C₃-C₈)-cycloalkyl, (C₆-C₁₄)-aryl or (C₃-C₁₃)-heteroaryl,
which, with the exception of the hydrogen, are in each case optionally monosubstituted by amino, substituted amino, amidino, substituted amidino, hydroxyl, carboxyl, carbamoyl, guanidino, substituted guanidino, ureido, substituted ureido, mercapto, methylmercapto, phenyl, 4-chlorophenyl, 4-fluorophenyl, 4-nitrophenyl, 4-methoxyphenyl, 4-hydroxyphenyl, phthalimido, 1,8-naphthalimido, 4-imidazolyl, 3-indolyl, 2-thienyl, 3-thienyl, 2-pyridyl, 3-pyridyl or cyclohexyl,
or in which
R(2) and R(3), together with the atoms carrying them, form a mono-, bi- or tricyclic ring system having from 2 to 15 carbon atoms;
A is as defined for P;
B is a basic amino acid in the L or D configuration, which may be substituted in the side chain;
C is a compound of the formula III a or III b 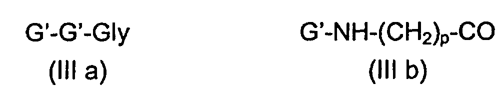 in which
p is from 2 to 8, and
G' independently of one another is a radical of the formula IV
-NR(4)-CHR(5)-CO- (IV)
in which
R(4) and R(5), together with the atoms carrying them, form a heterocyclic mono-, bi- or tricyclic ring system having from 2 to 15 carbon atoms;
E is the radical of a neutral, acidic or basic, aliphatic or alicyclicaliphatic amino acid;
F independently of one another is the radical of a neutral, acidic or basic, aliphatic or aromatic amino acid, which may be substituted in the side chain, or is a direct bond;
(D)Q is D-Tic, D-Phe, D-Oic, D-Thi or D-Nal, which may optionally be substituted by halogen, methyl or methoxy, or a radical of the following formula (V) 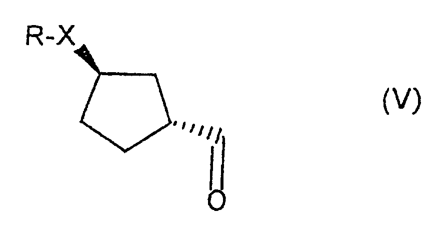 in which
X is oxygen, sulfur or a direct bond;
R is hydrogen, (C₁-C₈)-alkyl, (C₃-C₈)-cycloalkyl, (C₆-C₁₄)-aryl, (C₆-C₁₄)-aryl-(C₁-C₄)-alkyl, where cycloalkyl may optionally be substituted by halogen, methyl or methoxy;
G is as defined above for G' or is a direct bond;
F' is as defined for F, is a radical -NH-(CH₂)_{q}-, where q = 2 to 8, or, if G is not a direct bond, is a direct bond;
I is -OH, -NH₂ or NHC₂H₅, with the proviso that I is not bonded directly to (D)Q;
K is the radical -NH-(CH₂)ₓ-CO-, where x = 1 - 4, or a direct bond, and
M is as defined for F,
and physiologically tolerated salts thereof
for the preparation of medicaments for the treatment and prevention of Alzheimer's disease.

2. Use of a bradykinin antagonist of the formula I according to Claim 1, in which
Z is hydrogen or is as defined under a₁), a₂) or a₃),
P is a bond or a radical of the formula II
- NR(2) -(U)- CO - (II)
where U is CHR(3) and
R(3) is as defined above; or
in which R(2) and R(3), together with the atoms carrying them, form a mono-, bi- or tricyclic ring system having from 2 to 15 carbon atoms,
R(2) is H or CH₃, and
A is a bond.

3. Use of a bradykinin antagonist of the formula I according to Claim 1, in which
Z is hydrogen or is as defined under a₁), a₂) or a₃),
P is a bond or a radical of the formula II
- NR(2) -(U)- CO - (II)
where U is CHR(3), and R(3), independently of one another, is hydrogen, (C₁-C₆)-alkyl, (C₃-C₈)-cycloalkyl, (C₆-C₁₄)-aryl, (C₃-C₁₃)-heteroaryl, which, with the exception of the hydrogen, are each optionally monosubstituted by amino, substituted amino, hydroxyl, carboxyl, carbamoyl, guanidino, substituted guanidino, ureido, mercapto, methylmercapto, phenyl, 4-chlorophenyl, 4-fluorophenyl, 4-nitrophenyl, 4-methoxyphenyl, 4-hydroxyphenyl, phthalimido, 4-imidazolyl, 3-indolyl, 2-thienyl, 3-thienyl, 2-pyridyl, 3-pyridyl or cyclohexyl, or
in which R(2) and R(3), together with the atoms carrying them, form a mono-, bi- or tricyclic ring system having from 2 to 15 carbon atoms;
R(2) is H or CH₃,
A is a bond, and
(D)Q is D-Tic.

4. Use of a bradykinin antagonist according to Claims 1 - 3, **characterized in that** the bradykinin antagonist is
H-D-Arg-Arg-Pro-Hyp-Gly-Thi-Ser-D-Tic-Oic-Arg-OH (HOE 140), para-guanidinobenzoyl-Arg-Pro-Hyp-Gly-Thi-Ser-D-Tic-Oic-Arg-OH, H-D-Arg-Arg-Pro-Hyp-Gly-Phe-Ser-D-HypE(transpropyl)-Oic-Arg-OH, H-D-Arg-Arg-Pro-Hyp-Gly-Cpg-Ser-D-Cpg-Cpg-Arg-OH, H-D-Arg-Arg-Pro-Pro-Gly-Thi-Ser-D-Tic-Oic-Arg-OH, H-Arg(Tos)-Pro-Hyp-Gly-Thi-Ser-D-Tic-Oic-Arg-OH, H-Arg(Tos)-Pro-Hyp-Gly-Phe-Ser-D-Tic-Oic-Arg-OH, H-D-Arg-Arg-Pro-Hyp-Gly-Phe-Ser-D-Tic-Oic-Arg-OH, Fmoc-D-Arg-Arg-Pro-Hyp-Gly-Thi-Ser-D-Tic-Oic-Arg-OH, Fmoc-Aoc-D-Arg-Arg-Pro-Hyp-Gly-Thi-Ser-D-Tic-Oic-Arg-OH, Fmoc- -aminocaproyl-D-Arg-Arg-Pro-Hyp-Gly-Thi-Ser-D-Tic-Oic-Arg-OH, benzoyl-D-Arg-Arg-Pro-Hyp-Gly-Thi-Ser-D-Tic-Oic-Arg-OH, cyclohexylcarbonyl-D-Arg-Arg-Pro-Hyp-Gly-Thi-Ser-D-Tic-Oic-Arg-OH, Fmoc-Aeg(Fmoc)-D-Arg-Arg-Pro-Hyp-Gly-Thi-Ser-D-Tic-Oic-Arg-OH, Fmoc-Aeg(Fmoc)-Arg-Pro-Hyp-Gly-Thi-Ser-D-Tic-Oic-Arg-OH, indol-3-yl-acetyl-D-Arg-Arg-Pro-Hyp-Gly-Thi-Ser-D-Tic-Oic-Arg-OH or dibenzylacetyl-D-Arg-Arg-Pro-Hyp-Gly-Thi-Ser-D-Tic-Oic-Arg-OH.

5. Use of a bradykinin antagonist according to Claim 4, **characterized in that** the bradykinin antagonist is
H-D-Arg-Arg-Pro-Hyp-Gly-Thi-Ser-D-Tic-Oic-Arg-OH (HOE 140) or para-guanidobenzoyl-Arg-Pro-Hyp-Gly-Thi-Ser-D-Tic-Oic-Arg-OH.

6. Use of a bradykinin antagonist according to Claim 5, **characterized in that** the bradykinin antagonist is
H-D-Arg-Arg-Pro-Hyp-Giy-Thi-Ser-D-Tic-Oic-Arg-OH (HOE 140).

## Revendications

1. Utilisation d'un antagoniste de bradykinine de formule I
Z-P-A-B-C-E-F-K-(D)Q-G-M-F'-I (I)
dans laquelle :
Z représente
a₁) un atome d'hydrogène, un groupe alkyle en C₁-C₈, alcanoyle en C₁-C₈, alcoxy(C₁-C₈)carbonyle, cycloalkyle en C₃-C₈, cycloalcanoyle en C₄-C₉ ou alkyl(C₁-C₈)sulfonyle,
dans lesquels 1,2 ou 3 atomes d'hydrogène de chaque radical alkyle ou cycloalkyle précité sont éventuellement remplacés, respectivement, par 1, 2 ou 3 radicaux, identiques ou différents, choisis dans l'ensemble comprenant les groupes suivants :
carboxy, NHR(1), [alkyl(C₁-C₄)]NR(1) ou [aryl(C₆-C₁₀)-alkyl(C₁-C₄)]NR(1), R(1) représentant un atome d'hydrogène ou un groupe protecteur uréthanne, alkyle en C₁-C₄, alkyl(C₁-C₈)amino, aryl(C₆-C₁₀)-alkyl(C₁-C₄)-amino, hydroxy, alcoxy en C₁-C₄, halogéno, dialkyl-(C₁-C₈)amino, di-[aryl(C₆-C₁₀)-alkyl(C₁-C₄)]amino, carbamoyle, phtalimido, 1,8-naphtalimido, sulfamoyle, alcoxy(C₁-C₄)carbonyle, aryle(C₆-C₁₄) et aryl(C₆-C₁₄)-alkyle(C₁-C₅),
ou dans lesquels un atome d'hydrogène est éventuellement remplacé par un radical choisi dans l'ensemble constitué par les groupes suivants :
cycloalkyle en C₃-C₈, alkyl(C₁-C₆)sulfonyle, alkyl(C₁-C₆)-sulfinyle, aryle(C₆-C₁₄)-alkyl(C₁-C₄)sulfonyle, aryl-(C₆-C₁₄)-alkyl(C₁-C₄)sulfinyle, aryle en C₆-C₁₄, aryloxy en C₆-C₁₄, hétéroaryle en C₃-C₁₃ et hétéroaryl(C₃-C₁₃)-oxy
et 1 ou 2 atomes d'hydrogène sont remplacés par 1 ou 2 radicaux, identiques ou différents, choisis dans l'ensemble constitué par les groupes
carboxy, amino, alkyl(C₁-C₈)amino, hydroxy, alcoxy en C₁-C₄, halogéno, dialkyl(C₁-C₈)amino, carbamoyle, sulfamoyle, alcoxy(C₁-C₄)carbonyle, aryle en C₆-C₁₄ et aryl(C₆-C₁₄)-alkyle(C₁-C₅);
a₂) aryle en C₆-C₁₄, aroyle en C₇-C₁₅, aryl(C₆-C₁₄)sulfonyle, hétéroaryle en C₃-C₁₃ ou hétéroaroyle en C₃-C₁₃ ;
a₃) carbamoyle, qui peut éventuellement être substitué sur l'atome d'azote par un groupe alkyle en C₁-C₈, aryle en C₆-C₁₄ ou aryl(C₆-C₁₄)-alkyle(C₁-C₅) ;
dans les radicaux définis en a₁), a₂) et a₃), les groupes aryle, hétéroaryle, aroyle, arylsulfonyle et hétéroaroyle étant éventuellement substitués par 1, 2, 3 ou 4 radicaux choisis dans l'ensemble constitué par les groupes
carboxy, amino, nitro, alkyl(C₁-C₈)amino, hydroxy, alkyle en C₁-C₆, alcoxy en C₁-C₆, aryle en C₆-C₁₄, aroyle en C₇-C₁₅, halogéno, cyano, dialkyl(C₁-C₈)amino, carbamoyle, sulfamoyle et alcoxy(C₁-C₆)carbonyle ;
P représente une liaison directe ou un radical de formule II,
-NR(2)-(U)-CO- (II)
dans laquelle
R(2) représente un atome d'hydrogène, le groupe méthyle ou un groupe protecteur uréthanne,
U représente un groupe cycloalkylidène en C₃-C₈, arylidène en C₆-C₁₄, hétéroarylidène en C₃-C₁₃, aryl(C₆-C₁₄)-alkylidène(C₁-C₆), qui peuvent éventuellement être substitués, ou [CHR(3)]ₙ, n étant égal à 1-8, de préférence 1-6 et
les radicaux R(3) représentant, indépendamment les uns des autres, un atome d'hydrogène ou un groupe alkyle en C₁-C₆, cycloalkyle en C₃-C₈, aryle en C₆-C₁₄ ou hétéroaryle en C₃-C₁₃, qui, à l'exception de l'atome d'hydrogène, sont éventuellement monosubstitués chacun par un groupe amino, amino substitué, amidino, amidino substitué, hydroxy, carboxy, carbamoyle, guanidino, guanidino substitué, uréido, uréido substitué, mercapto, méthylmercapto, phényle, 4-chlorophényle, 4-fluorophényle, 4-nitrophényle, 4-méthoxyphényle, 4-hydroxyphényle, phtalimido, 1,8-naphtalimido, 4-imidazolyle, 3-indolyle, 2-thiényle, 3-thiényle, 2-pyridyle, 3-pyridyle ou cyclohexyle,
ou dans laquelle
R(2) et R(3) forment ensemble, avec les atomes qui les portent, un système cyclique mono-, bi- ou tricyclique ayant de 2 à 15 atomes de carbone ;
A est défini comme P ;
B représente un aminoacide basique en configuration L ou D, qui peut être substitué sur la chaîne latérale ;
C représente un composé de formule IIIa ou IIIb 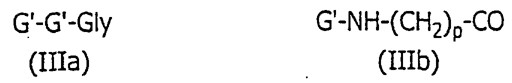
dans lesquelles
p vaut 2 à 8, et
les radicaux G' représentent, indépendamment les uns des autres, un radical de formule IV
-NR(4)-CHR(5)-CO (IV)
dans laquelle
R(4) et R(5) forment ensemble, avec les atomes qui les portent, un système hétérocyclique mono-, bi- ou tricyclique ayant de 2 à 15 atomes de carbone ;
E représente le reste d'un aminoacide aliphatique ou alicyclique-aliphatique neutre, acide ou basique ;
F représente le reste d'un aminoacide aliphatique ou aromatique neutre, acide ou basique, qui peut être substitué sur la chaîne latérale, ou une liaison directe ;
(D)Q représente D-Tic, D-Phe, D-Oic, D-Thi ou D-Nal, qui peuvent éventuellement être substitués par des halogènes ou le groupe méthyle ou méthoxy, ou un radical de formule (V) suivante 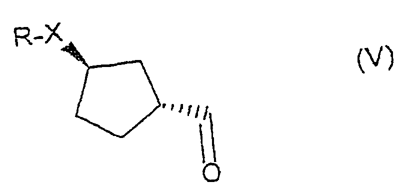 dans laquelle
X représente un atome d'oxygène, de soufre ou une liaison directe ;
R représente un atome d'hydrogène ou un groupe alkyle en C₁-C₈, cycloalkyle en C₃-C₈, aryle en C₆-C₁₄, aryl(C₆-C₁₄)-alkyle(C₁-C₄), le radical cycloalkyle pouvant éventuellement être substitué par un atome d'halogène ou par le groupe méthyle ou méthoxy ;
G est défini comme G' plus haut ou représente une liaison directe ;
F' est défini comme F, ou représente un radical -NH-(CH₂)_{q}-, dans lequel q = 2 à 8, ou représente une liaison directe lorsque G ne représente pas une liaison directe ;
I représente -OH, -NH₂ ou NHC₂H₅, étant entendu que I n'est pas lié directement à (D)Q ;
K représente le radical -NH-(CH₂)ₓ-CO-, dans lequel x = 1-4, ou une liaison directe, et
M est défini comme F,
ainsi que leurs sels physiologiquement acceptables,
pour la fabrication de médicaments destinés au traitement et à la prévention de la maladie d'Alzheimer.

2. Utilisation d'un antagoniste de bradykinine de formule I selon la revendication 1, dans laquelle
Z représente un atome d'hydrogène ou est tel que défini plus haut en a₁), a₂) ou a₃),
P représente une liaison ou un radical de formule II
-NR(2)-(U)-CO- (II)
dans laquelle U représente CHR(3) et R(3) est tel que défini plus haut ; ou
dans laquelle R(2) et R(3) forment ensemble, avec les atomes qui les portent, un système cyclique mono-, bi- ou tricyclique ayant de 2 à 15 atomes de carbone, R(2) représente H ou CH₃,
A représente une liaison.

3. Utilisation d'un antagoniste de bradykinine de formule I selon la revendication 1, dans lequel
Z représente un atome d'hydrogène ou est tel que défini plus haut en a₁), a₂) ou a₃),
P représente une liaison ou un radical de formule II
-NR(2)-(U)-CO- (II)
dans laquelle U représente CHR(3) et
R(3) représente un atome d'hydrogène ou un groupe alkyle en C₁-C₆, cycloalkyle en C₃-C₈, aryle en C₆-C₁₄, hétéroaryle en C₃-C₁₃, qui, à l'exception de l'atome d'hydrogène, sont éventuellement monosubstitués chacun par
un groupe amino, amino substitué, hydroxy, carboxy, carbamoyle, guanidino, guanidino substitué, uréido, mercapto, méthylmercapto, phényle, 4-chlorophényle, 4-fluorophényle, 4-nitrophényle, 4-méthoxyphényle, 4-hydroxyphényle, phtalimido, 4-imidazolyle, 3-indolyle, 2-thiényle, 3-thiényle, 2-pyridyle, 3-pyridyle ou cyclohexyle, ou
dans laquelle R(2) et R(3) forment ensemble, avec les atomes qui les portent, un système cyclique mono-, bi- ou tricyclique ayant de 2 à 15 atomes de carbone,
R(2) représente H ou CH₃ ;
A représente une liaison ;
D(Q) représente D-Tic.

4. Utilisation d'un antagoniste de bradykinine de formule I selon les revendications 1 à 3, **caractérisé en ce que** l'antagoniste de bradykinine est
H-D-Arg-Arg-Pro-Hyp-Gly-Thi-Ser-D-Tic-Oic-Arg-OH (HOE 140) para-guanidobenzoyl-Arg-Pro-Hyp-Gly-Thi-Ser-D-Tic-Oic-Arg-OH H-D-Arg-Arg-Pro-Hyp-Gly-Phe-Ser-D-HypE(transpropyl)-Oic-Arg-OH H-D-Arg-Arg-Pro-Hyp-Gly-Cpg-Ser-D-Cpg-Cpg-Arg-OH H-D-Arg-Arg-Pro-Pro-Gly-Thi-Ser-D-Tic-Oic-Arg-OH H-Arg(Tos)-Pro-Hyp-Gly-Thi-Ser-D-Tic-Oic-Arg-OH H-Arg(Tos)-Pro-Hyp-Gly-Phe-Ser-D-Tic-Oic-Arg-OH H-D-Arg-Arg-Pro-Hyp-Gly-Phe-Ser-D-Tic-Oic-Arg-OH Fmoc-D-Arg-Arg-Pro-Hyp-Gly-Thi-Ser-D-Tic-Oic-Arg-OH Fmoc-Aoc-D-Arg-Arg-Pro-Hyp-Gly-Thi-Ser-D-Tic-Oic-Arg-OH Fmoc-ε-aminocaproyl-D-Arg-Arg-Pro-Hyp-Gly-Thi-Ser-D-Tic-Oic-Arg-OH benzoyl-D-Arg-Arg-Pro-Hyp-Gly-Thi-Ser-D-Tic-Oic-Arg-OH cyclohexylcarbonyl-D-Arg-Arg-Pro-Hyp-Gly-Thi-Ser-D-Tic-Oic-Arg-OH Fmoc-Aeg(Fmoc)-D-Arg-Arg-Pro-Hyp-Gly-Thi-Ser-D-Tic-Oic-Arg-OH Fmoc-Aeg(Fmoc)-Arg-Pro-Hyp-Gly-Thi-Ser-D-Tic-Oic-Arg-OH indol-3-ylacétyl-D-Arg-Arg-Pro-Hyp-Gly-Thi-Ser-D-Tic-Oic-Arg-OH ou dibenzylacétyl-D-Arg-Arg-Pro-Hyp-Gly-Thi-Ser-D-Tic-Oic-Arg-OH.

5. Utilisation d'un antagoniste de bradykinine selon la revendication 4, **caractérisé en ce que** l'antagoniste de bradykinine est
H-D-Arg-Arg-Pro-Hyp-Gly-Thi-Ser-D-Tic-Oic-Arg-OH (HOE 140) ou para-guanidobenzoyl-Arg-Pro-Hyp-Gly-Thi-Ser-D-Tic-Oic-Arg-OH.

6. Utilisation d'un antagoniste de bradykinine selon la revendication 5, **caractérisé en ce que** l'antagoniste de bradykinine est
H-D-Arg-Arg-Pro-Hyp-Gly-Thi-Ser-D-Tic-Oic-Arg-OH (HOE 140).
